Europäisches Patentamt

European Patent Office

Office européen des brevets

(11) Publication number: **0 126 321**
**B1**

# EUROPEAN PATENT SPECIFICATION

(45) Date of publication of patent specification: **07.12.88**

(51) Int. Cl.⁴: **A 61 M 16/00**

(21) Application number: **84104609.7**

(22) Date of filing: **25.04.84**

(54) Demand valve for a breathing apparatus.

(30) Priority: **19.05.83 IT 2117783**

(43) Date of publication of application:
**28.11.84 Bulletin 84/48**

(45) Publication of the grant of the patent:
**07.12.88 Bulletin 88/49**

(84) Designated Contracting States:
**DE FR GB SE**

(56) References cited:
**GB-A-1 401 613**
**US-A-3 360 007**
**US-A-3 866 622**

(73) Proprietor: **SEKUR S.p.A.**
**Piazzale Cadorna, 5**
**I-20123 Milan (IT)**

(72) Inventor: **Cappa, Giulio**
**Viale Tunisia, 39**
**Milan (IT)**
Inventor: **Moscatelli, Romano**
**Via Massaciuccoli, 12**
**Roma (IT)**

(74) Representative: **Mariani, Giorgio, Dr. et al**
**INDUSTRIE PIRELLI S.p.A. Direzione Brevetti**
**Piazzale Cadorna, 5**
**I-20123 Milano (IT)**

Courier Press, Leamington Spa, England.

## Description

The present invention refers to a demand valve for introducing breathable air into a self-breathing apparatus (compressed air breathing apparatus)—in particular, inside the mask of the self-breathing apparatus itself.

The distributor, along with which the commutator device of the invention can be utilized, is of the type according to GB—A—1.401.613 that comprises a transducer for controlling the movement of a valve element for feeding predetermined quantities of air into a chamber of the distributor itself, following a pressure variation that is verified in said chamber.

The above-said transducer comprises substantially, a deformable membrane upon one wall of which there rests a stylus, that is made fast with a rod connected to the previously mentioned shutter-element—in such a way that, following a deformation of the membrane, the position of said shutter-element is varied.

One wall of said membrane also constitutes one of the walls that delimit the above said chamber—and hence, on one of its surfaces, there acts the pressure that exists inside the chamber itself, whilst on the other surface of the same wall, atmospheric pressure is caused to act.

The distributors of the type described are apt for being utilized substantially in two different configurations.

In a first of these configurations, the above-mentioned transducer is predisposed for controlling the opening of the shutter-element for the purpose of feeding air into the above-said chamber only when the pressure inside it results as being lower than atmospheric pressure.

For predisposing the distributor for functioning as in said first configuration, it is necessary that, on a first surface of the previously-mentioned membrane wall (that also constitutes one of the surfaces which delimit the said chamber), there acts the pressure existing inside the chamber itself, which atmospheric pressure acts on a second surface of the same wall.

In this way, said wall is equilibrated between the two pressures—and hence, it is able to shift whenever the pressure, inside chamber, descends to below atmospheric pressure.

For predisposing the distributor for functioning as in the second configuration, it is necessary that, on said first surface, there is also caused to act a predetermined force generated by a spring.

Under these conditions, the membrane can shift for controlling the opening of the shutter-element, only when the pressure inside the chamber is higher than atmospheric pressure and when the elastic reaction applied by the spring itself is higher than the pressure inside the chamber.

On the basis of the present invention, there is realized a commutator device for an air distributor, of the type described, which results as being structurally very simple, which can be set-up and dismantled with considerable facility, and whose functioning is safe and reliable.

On the basis of the present invention, there is realized a demand valve for a breathing apparatus, comprising a transducer for controlling the movements of a valve element for feeding predetermined quantities of air into a chamber in response to a pressure variation inside said chamber, said transducer being activated in response to movement, of a deformable membrane exposed to the pressure which exists in said chamber, said device being apt for switching said distributor from a first configuration—wherein, on a first surface of said membrane, there acts the pressure existing inside said chamber, and wherein, on a second surface of the membrane, the atmospheric pressure acts, into a second-configuration wherein, on said second surface, a predetermined force, generated by a spring, is also caused to act, characterized by the fact that said spring is disposed inside a piston which bears against said second surface of said membrane and which is axially mobile inside a cylindrical portion of a cover of the casing of said distributor, said piston being provided with a plurality of radial projections, each of which bears against a corresponding cam surface having a helicoidal profile formed on the lateral surface of said cylindrical portion, with manually operable actuating means for controlling the rotation of said piston about its own axis—and hence, the axial movement of said piston in the spring compressing sense, for switching said distributor from said second configuration into said first configuration, said actuating means comprises a rotatable sleeve, co-axial with said piston and interposed between the latter and said cylindrical portion of the cover, said sleeve being provided with axial slits, inside each of which there is inserted one of said projections, and with an actuating-handle that protrudes above said cover, and said demand valve comprises stopping means for limiting the rotation of the sleeve with respect to said cover between two predetermined angular positions, that are arranged at the angle which is necessary for rotating said sleeve, for moving said piston from the axial position in which it is found in said second configuration into the axial position in which it is found in said first configuration.

For having a better understanding of the device of the invention, there will now be given, by way of example, the description of one of its particular forms of realization, with referring to the attached drawings, whereby:—

Fig. 1 shows a plan view of the distributor, in which there is seen incorporated the commutator device of the present invention;

Fig. 2 shows a cross-section of the distributor of Fig. 1, effectuated with a marked out plane II—II;

Fig. 3 shows a plan view of the distributor of Fig. 1, in a diverse operative configuration;

Fig. 4 shows a cross-section of the distributor of Fig. 3, effectuated with a marked out plane III—III;

Fig. 5 shows a perspective view, in pieces, apt for illustrating the form of the main elements of the device of the invention;

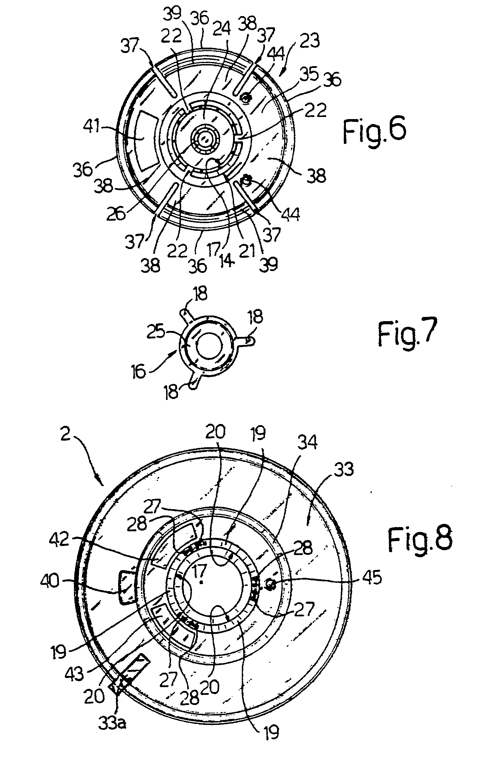

Fig. 6 shows a plan view, seen from below, of a sleeve that forms part of the device of the invention;

Fig. 7 shows a plan view of a small-piston that forms part of this device;

Fig. 8 shows a plan view seen from above, of a cover of the distributor, in which the device is incorporated;

Figs. 9 and 10 show two partial sections of the device, apt for pointing out the modalities for dismantling the device itself.

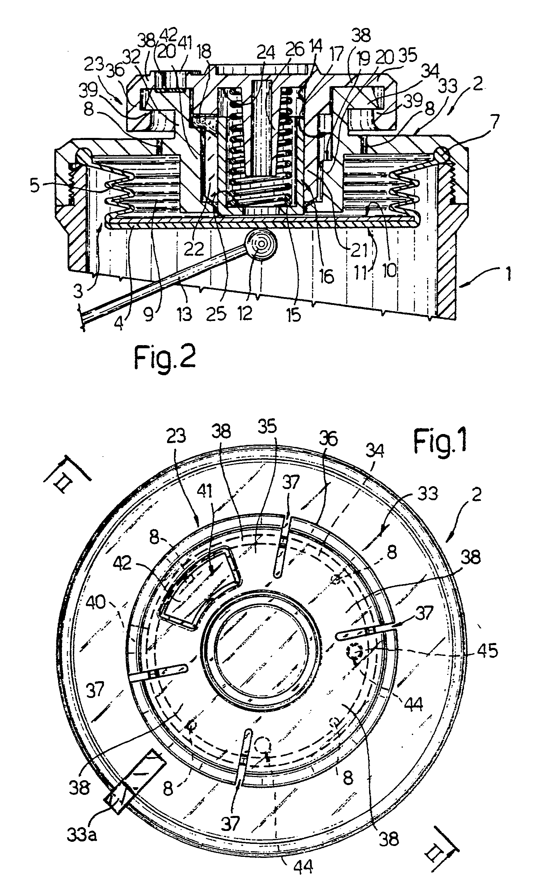

First and foremost, with reference to Figs. 1 and 2, the commutator device of the invention is destined for an air-distributor—of the type that can be seen partially in Figs. 1 and 2, which comprises a casing 1 and a cover 2, connectable, in any whatsoever convenient way, to the casing itself, for defining inside them a substantially cylindrical chamber 3. The distributor comprises moreover, a deformable membrane provided with a substantially flat wall 4 and of a lateral substantially cylindrical wall 5 that is provided with a series of corrugations for rendering it axially deformable. The circular edge 7 of said membrane is blocked between the cover 2 and the casing 1—in the manner visible in Fig. 2.

A series of holes 8, made in the cover 2, put into communication the atmosphere with an internal cavity 9 defined within the membrane itself. With said constructive disposition, the atmospheric pressure acts on the surface 10 of the wall 4 of the membrane; while on the other surface 11 of the wall itself, there acts the pressure existing inside the chamber 3.

A transducer is apt for controlling the opening of the valve-element (not shown) for feeding air into the chamber 3. The air is furnished by a source of compressed-air. Said transducer substantially comprises a stylus 12, resting on the surface 11 of the membrane-wall 4, and an actuating rod 13 that is connected with the above-said valve-element, in such a way that—following an axial shifting of the wall 4, the actuating rod 13 is caused to pivot for varying the passage-hole controlled by the valve-element.

The described distributor is predisposed for functioning in two different configurations. In the first one of these, on the surfaces 10 and 11 of the membrane-wall 4, there only act respectively, the atmospheric pressure and the pressure which exists inside the chamber 3. Hence, in this configuration, with the wall 4 being equilibrated between the above-said two pressures, the opening of the valve-element is controlled for allowing the entry of the air into the chamber itself, when the pressure inside it is below that of atmospheric pressure (negative pressure). The distributor is moreover in a condition for functioning in a second-configuration—wherein on the surface 10 of the wall 4, there is also caused to act the force exercised by a spiral-spring 15, which is interposed (in a manner to be explained further on in the text) between the cover 2 and the membrane itself. The above-said spring exercises said axial force on the wall 4, through a small piston 16, that is axially mobile with respect to the cover 2.

Therefore, in said second, operative configuration, since on the wall 4 there act—apart from the pressures existing in the chambers 9 and 3, also the forces exercised by the spring 15, the opening of the valve-evement is controlled whenever the pressure inside the chamber 3—although still remaining higher than atmosphere pressure (positive pressure), descends to below a predetermined value (equal to the elastic reaction of the spring divided by the frontal surface 11 of the membrane).

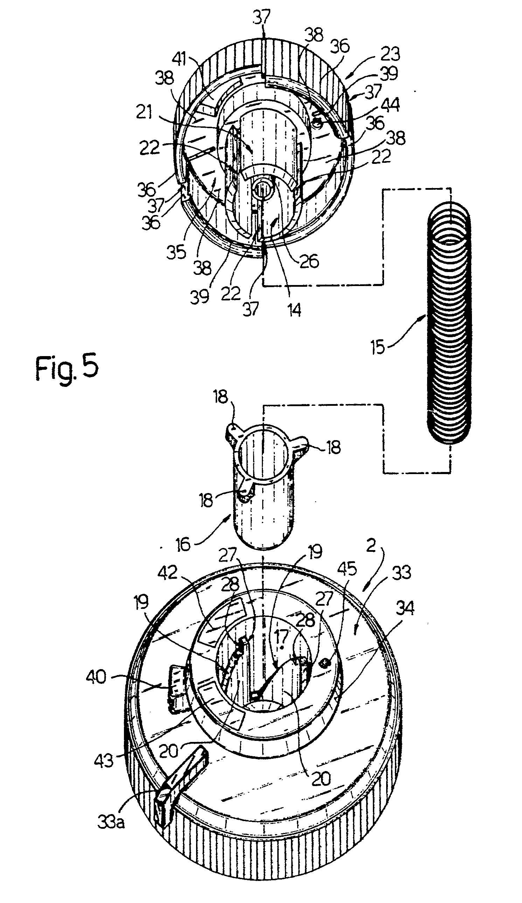

On the basis of the invention, the small piston 16, is provided with a plurality of radial stakes 18—each one of which is apt for bearing on the active surface 19 (Fig. 5) of a corresponding cam 20, which presents a substantially helicoidal profile and which is made on the internal surface of a hole 17 of the cover 2. The device comprises moreover, a rotatable sleeve 21—which is interposed between said surface and the small piston 16. Said sleeve presents a plurality of axial slits 22 (Fig. 5)—in each one of which there is slidingly inserted one of the stakes 18 (as can be seen clearly in Fig. 2). The said sleeve is moreover, provided with an actuating-handle 23—made all in one-piece with the sleeve itself, apt for controlling the rotation of said sleeve.

An annular surface 24 (Fig. 2), made inside the sleeve 21, constitutes a resting-surface for the spring 15, which is interposed between said surface and another resting-surface 25 made inside the small piston 16. An appendix 26 of the handle 23, protrudes axially inside the sleeve 21, and it is inserted into the spring itself.

In the instance of the form of realization described, inside the hole 17 there are made three cams 20—having a phase-displacement angle of 120° substantially, with each of them (as can clearly be seen in Fig. 5) comprising a substantially helicoidal active-surface 19 that is coaxial with the axis of the hole 17 and predisposed for constituting a supporting-surface for a corresponding stake 18 of the small-piston 16, and a corresponding pair of substantially axial stopping-surfaces 27 and 28, disposed laterally to said active-surface, apt for delimiting the rotation of the small-piston itself with respect to the cover, in a way that shall be explained further on in the text.

The cover 2 comprises moreover, an annular-relief 32, that protrudes axially above the surface 33 of the cover itself, and which is provided with a substantially annular-rim 34. The handle 23 presents a first wall 35, that is substantially flat and circular, apt for superimposing the annular-relief 32 having an annular-rim 34, as well as a second cylindrical wall 36 that is disposed in the outermost peripheral position with respect to the annular-rim 34—as is clearly seen in Fig. 2.

For convenience sake, in the first and second walls 35 and 36 of the handle, there are made radial grooves 37 that are apt for defining the sectors of the handle 38 (Fig. 1), and the cylindrical wall 36, of each one of these sectors, is provided with radial teeth 39 (Fig. 2), apt for cooperating with the annular-rim 34—in such a way as to allow a spring-fitting of the handle to the cover.

From the annular-relief 32, there protrudes radially a pad 40 apt for coming into contact with the stopping-teeth 39 of the sectors 38, for limiting the end-of-rotation of the handle 23 with respect to the cover 2, between two prefixed angular positions—which are out of phase with each other, at a suitable angle.

An aperture 41 is made in the wall 35 of the handle 23. Said aperture—when said handle is taken to one of the two above-mentioned angular positions, is apt for uncovering a corresponding surface-of-reference 42 or 43, made on the annular-rim 34.

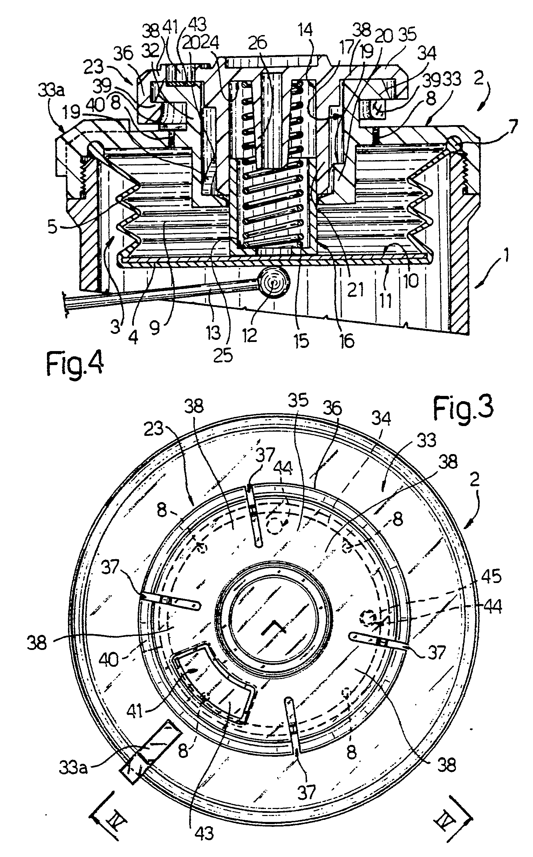

Moreover, for convenience sake, a radial relief 33a is made on the cover 2, above the upper surface 33 of the cover itself. This relief is aligned with the surface-of-reference 43—as can clearly be seen in Fig. 3.

On the lower surface of the wall 35 of the cover 23 moreover, there is made a pair of cavities 44 (see Fig. 5), each one of which is apt for co-operating with a corresponding protuberance 45 made on the rim 34—in such a way as to determine a spring-stop, whenever one of the two above-mentioned angular positions is reached.

The functioning of the commutator device of the invention takes place in the following manner.

Let us suppose that the commutator device has been set up in such a way as to be disposed in the previously defined first-configuration, i.e. on whose surfaces 10 and 11, of the membrane wall 4, there only act the atmospheric pressure and the pressure existing inside the chamber 3. In this configuration, the handle 23 (Fig. 1) has been completely rotated in the clockwise direction (shown in Fig. 1), till when the teeth 39 of one of the sectors 38 was made to stop against the pad 40 (Fig. 5). In the same configuration, the aperture 41 uncovers the surface-of-reference 42 (Fig. 1), in such a way as to furnish a visual identification of the configuration wherein the commutator device is found.

When the handle 23 has been rotated in the above-indicated manner, the slits 22, of the sleeve 21, are found in an angular position with respect to the cover 2, in such a way as to carry the stakes 18 of the small-piston 16 for being disposed substantially on the initial, more raised part, of the active-surface 19 (Figs. 2 and 5) of the cam 20 (as has been shown in Fig. 2). In this configuration, the small piston 16 results as being completely inside the hole 14 of the sleeve 21 (as can be clearly seen in Fig. 2) and hence, it does not exercise any action upon the surface 10 of the membrane wall 4. In said configuration, each stake 18, becomes stopped by the corresponding blocking-surface 28 (Fig. 5)—that delimits, from one side, each active-surface 19 of the cam 20.

When the commutator device must be taken to the second-configuration—wherein on the surface 10 of the wall 4, there is also made to act that force exercised by the spring 15, the handle 23 becomes rotated in the anti-clockwise direction (Fig. 1)—for taking it to the configuration shown in Fig. 3, in which the teeth 39 of one of the sectors 38 becomes stopped against the pad 40 (Fig. 5). In the same configuration, the aperture 41 uncovers the surface-of-reference 43 that is apt for furnishing a visual indication of the new configuration assumed by the device.

In the same configuration, the aperture 41 is aligned radially with the relief 33a, and hence, in association with the relief itself, it is apt for furnishing a tactile indication of the configuration wherein the device is set up.

Following to the rotation of the handle 23, the sleeve 21 causes the stakes 18 of this small-piston 16, to rotate. Said piston—under the thrust of spring 15, moves into the hole 14, while the stakes 18 are guided by the active-surface 19 of the cam 20. At the end of the above-said rotation, the small-piston 16 can go and rest on the surface 10 of the membrane wall 4 (as is shown in Fig. 4), in such a way as to exercise upon it the force generated by the spring 15. It is evident that—owing to their conformation, the cams 20 realize a substantially helicoidal-bond of the unilateral type, and the small-piston 16 can thereupon assume any whatsoever relative axial position, with respect to the hole 14, depending upon the position of the membrane wall 4.

When the device has once again to be commutated into the first-configuration, the handle 23 is rotated in the anti-clockwise sense (Fig. 3), and thereupon, the sleeve 21—owing to the coupling of the slits 22 with the stakes 18 of the small-piston 16, causes these stakes to rotate with forcing them to follow the profile—as as result of said rotation and owing to the effect of the coupling with the active-surface 19 of the cams 20, with again taking the small-piston 16 into the initial position of Fig. 2.

The cavities 44 and the protuberances 45, are positioned in such a way that one of the former become coupled with the latter, when the handle 23 is set up in one of the two configurations shown in Figs. 1 or 3, in such a way as to create a spring-action in correspondence of achieving the configurations themselves.

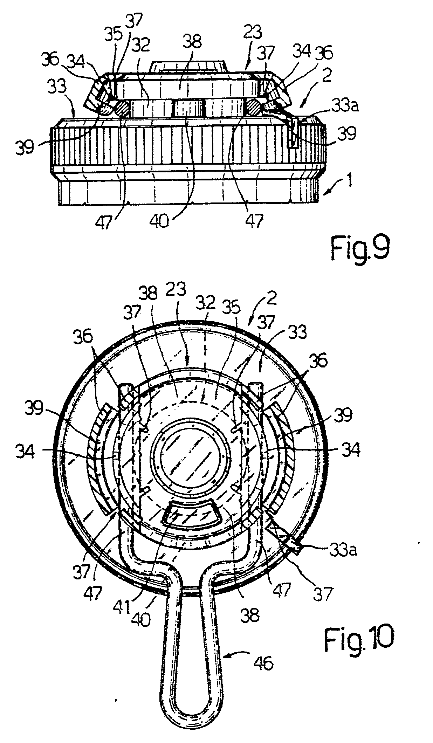

The dismantling of the device described, can take place in a very simple way—as shown in the Figs. 9 and 10. To this purpose, it is sufficient to rotate the handle 23 into an intermediate position—between those of Figs. 1 and 3—i.e. in which the aperture 41 is disposed approximately in correspondence of the pad 40, as has been shown in Fig. 10. By successively introducing a simple implement 46—provided with a pair of substantially parallel arms, between the handle 23 and the cover 22, it is possible to determine the widening of the lateral walls 36 of the handle itself (as is clearly shown in Fig. 9), in such a way as to release the teeth 39 of the sectors 38 from the corresponding annular-rim 34. Under these conditions, by exercising, with the implement itself, a certain axial force that tends to detach the handle 23 from the cover 2, the former can easily be separated from the latter.

For mounting the device, it is sufficient—after having inserted the spring 15 and the small-piston 16 into the hole 14 of the handle 23, to introduce the sleeve 21 into the hole 17 of the cover 2, and to successively exercise a small axial force on the handle 23, in such a way as to elastically deform the lateral walls 36 of the sectors 38, for causing the teeth 39 to jump below the annular rim 34.

Hence, it results as being evident that the commutator device of the invention can be considered very simple to mount, with its comprising few parts—that can be constructed with facility by utilizing thermoplastic materials and the usual technology of pressing and injections. Even the mounting of the various parts of the device, results as being rapid and easy. The functioning of this device is moreover, absolutely reliable and secure.

What is evident is that, to the form of realization of the present invention which has been described herein, there can also be carried out modifications and variations—both in the form, as well as in the disposition of the various parts, without departing from the ambit of the invention as defined in the claims.

## Claims

1. Demand valve for a breathing apparatus, comprising a transducer for controlling the movements of a valve element for feeding predetermined quantities of air into a chamber (3) in response to a pressure variation inside said chamber, said transducer being activated in response to movement of a deformable membrane exposed to the pressure which exists in said chamber, said device being apt for switching said distributor from a first configuration—wherein, on a first surface (11) of said membrane, there acts the pressure existing inside said chamber, and wherein, on a second surface (10) of the membrane, the atmospheric pressure acts, into a second configuration wherein, on said second surface, a predetermined force, generated by a spring (15), is also caused to act, characterized by the fact that said spring is disposed inside a piston (16) which bears against said second surface of said membrane and which is axially mobile inside a cylindrical portion of a cover of the casing of said distributor, said piston being provided with a plurality of radial projections (18), each of which bears against a corresponding cam surface (20) having a helicoidal profile formed on the lateral surface of said cylindrical portion (17), with manually operable actuating means for controlling the rotation of said piston about its own axis—and hence, the axial movement of said piston in the spring compressing sense, for switching said distributor from said second configuration into said first configuration, said actuating means comprises a rotatable sleeve (21), co-axial with said piston and interposed between the latter and said cylindrical portion of the cover, said sleeve being provided with axial slits (22), inside each of which there is inserted one of said projections, and with an actuating-handle (23) that protrudes above said cover, and said demand valve comprises stopping means (39, 40) for limiting the rotation of the sleeve (21) with respect to said cover (2) between two predetermined angular positions, that are arranged at the angle which is necessary for rotating said sleeve, for moving said piston from the axial position in which it is found in said second configuration into the axial position in which it is found in said first configuration.

2. Demand valve according to Claim 1, characterized by the fact that said sleeve is provided with a bottom base-wall (24) which acts as a support for said spring, and with an appendix (26), protruding axially from said base-wall, positioned within said spring, for axially centering it.

3. Demand valve according to any of the preceding claims, characterized by the fact that said cover comprises an annular-relief (32), coaxial with said hole, disposed around it, and an annular rim (34) extending radially outwardly from said annular relief, said handle of said element being provided with a first planar wall (35) which rests upon said annular relief and upon said annular rim, and with a second cylindrical wall (36), peripherically outside said annular rim, and disposed around it, at least a part of said second cylindrical wall being provided with elastic fixing means, inserted spring wise on said annular rim, for axially securing said handle with respect to the annular rim.

4. Demand valve according to Claim 3, characterized by the fact that said elastic fixing means are realized by means of radial cuts (37), made on said first and second walls of the handle, which render elastically deformable the sector (38) of said handle, which is situated between two contiguous cuts, and through radial teeth (39) provided on the inner surface of said second wall which engage said annular rim.

5. Demand valve according to Claim 1, characterized by the fact that said stopping-means comprises radial protuberances (40) provided on said annular relief of the cover for co-operating with said radial teeth (39) of said second handle wall.

6. Demand valve according to any of Claims 3 to 5, characterized by the fact that on said first handle wall (35), there is provided at least one aperture (41) for uncovering parts of the surface of said annular relief and said annular rim for furnishing a visual indication of the angular position of the handle.

7. Demand valve according to Claim 6, characterized by the fact that said cover comprises an annular relief (33a) aligned with said aperture (41) for furnishing a tactile indication of said angular position.

## Patentansprüche

1. Sofortventil für ein Atmungsgerät, umfassend einen Wandler zum Steuern der Bewegungen eines Ventilelementes, um vorbestimmte Luftmengen in eine Kammer (3) beim Ansprechen auf eine Druckänderung in der Kammer zu führen, wobei der Wandler beim Ansprechen auf Bewegung einer verformbaren Membran aktiviert wird, die dem Druck ausgesetzt ist, der in der Kammer herrscht, und wobei eine Einrichtung vorgesehen ist zum Schalten eines Verteilers von einer ersten Konfiguration, in welcher an einer ersten Fläche (11) der Membran der in der Kammer herrschende Druck, und an einer zweiten Fläche (10) der Membran der Atmosphärendruck wirkt, zu einer zweiten Konfiguration, in welcher eine von einer Feder (15) erzeugte vorbestimmte Kraft ebenfalls veranlaßt wird, an der zweiten Fläche zu wirken, dadurch gekennzeichnet, daß die Feder in einem Kolben (16) angeordnet ist, der sich gegen die zweite Fläche der Membran bewegt und der in einem zylindrischen Teil einer Abdeckung des Gehäuses des Verteilers axial beweglich ist, der Kolben mit einer Mehrzahl von radialen Vorsprüngen (18) versehen ist, deren jeder sich gegen eine entsprechende Nockenfläche (20) legt, die ein Helikoidprofil hat und an der Seitenfläche des zylindrischen Teiles (17) gebildet ist, eine von Hand betätigbare Betätigungseinrichtung vorgesehen ist zum Steuern der Drehung des Kolbens um seine Achse und demgemäß der axialen Bewegung des Kolbens im Sinn des Zusammendrückens der Feder, um den Verteiler von der zweiten Konfiguration zu der ersten Konfiguration zu schalten, die Betätigungseinrichtung eine drehbare Hülse (21) aufweist, die gleichachsig zu dem Kolben und zwischen dem letzteren und dem zylindrischen Teil der Abdeckung angeordnet ist sowie mit axialen Schlitzen (22), wobei in jeden Schlitz einer der Vorsprünge eingesetzt ist, und mit einem Betätigungshandgriff (23) versehen ist, der über die Abdeckung vorragt, und das Sofortventil zum Begrenzen der Drehung der Hülse (21) mit Bezug auf die Abdeckung (2) zwischen zwei vorbestimmten Winkelpositionen eine Anhalteeinrichtung (39, 40) aufweist, die an dem Winkel angeordnet sind, der erforderlich ist, um die Hülse zu drehen für Bewegung des Kolbens aus der axialen Position, in der er sich in der zweiten Konfiguration befindet, in die axiale Position, die er in der ersten Konfiguration einnimmt.

2. Sofortventil nach Anspruch 1, dadurch gekennzeichnet, daß die Hülse mit einer Bodenbasiswand (24), die als Abstützung für die Feder dient, und mit einem Fortsatz (26) versehen ist, der von der Basiswand axial vorragt und in der Feder angeordnet ist, um diese axial zu zentrieren.

3. Sofortventil nach irgendeinem der vorhergehenden Ansprüche, dadurch gekennzeichnet, daß die Abdeckung eine ringförmige Vertiefung (32), die gleichachsig zu dem Gehäuse liegt, welches um sie herum angeordnet ist, und einen ringförmigen Rand (34) aufweist, der sich von der ringförmigen Vertiefung radial nach außen erstreckt, der Handgriff des Elementes mit einer ersten ebenen Wand (35), die auf der ringförmigen Vertiefung und auf dem ringförmigen Rand ruht, und mit einer zweiten zylindrischen Wand (36) versehen ist, die auf dem Umfang außerhalb des ringförmigen Randes angeordnet und rund um diesen vorgesehen ist, wenigstens ein Teil der zweiten zylindrischen Wand mit elastischen Befestigungsmitteln versehen ist, die nach Federart an dem ringförmigen Rand eingesetzt sind, um den Handgriff mit Bezug auf den ringförmigen Rand axial zu sichern.

4. Sofortventil nach Anspruch 3, dadurch gekennzeichnet, daß die elastischen Befestigungsmittel durch radiale Einschnitte (37), die an der ersten und der zweiten Wand des Handgriffes gebildet sind und den Abschnitt (38) des Handgriffs elastisch verformbar machen, der zwischen zwei aneinander angrenzenden Einschnitten angeordnet ist, und durch radiale Zähne (39) dargestellt sind, die an der Innenfläche der zweiten Wand vorgesehen sind, welche mit dem ringförmigen Rand im Eingriff steht.

5. Sofortventil nach Anspruch 1, dadurch gekennzeichnet, daß die Anhalteeinrichtung radiale Vorsprünge (40) aufweist, die an der ringförmigen Vertiefung der Abdeckung vorgesehen sind für Zusammenarbeit mit den radialen Zähnen (39) der zweiten Handgriffwand.

6. Sofortventil nach irgendeinem der Ansprüche 3 bis 5, dadurch gekennzeichnet, daß an der ersten Handgriffwand (35) wenigstens eine Öffnung (41) vorgesehen ist zum Freilegen der Fläche der ringförmigen Vertiefung und des ringförmigen Randes, um eine Sichtanzeige für die Winkelposition des Handgriffes zu liefern.

7. Sofortventil nach Anspruch 6, dadurch gekennzeichnet, daß die Abdeckung eine ringförmige Vertiefung (33a) aufweist, die mit der Öffnung (41) ausgerichtet ist, um eine Fühlanzeige der genannten Winkelposition zu liefern.

**Revendications**

1. Valve de demande d'air pour un appareil respiratoire, comprenant un transducteur pour commander les mouvements d'un élément obturateur de la valve afin de fournir des quantités prédéterminées d'air dans une chambre (3) en réponse à une variation de pression à l'intérieur de ladite chambre, ledit transducteur étant actionné en réponse à un mouvement d'une membrane déformable exposée à la pression existant dans ladite chambre, ledit dispositif pouvant commuter ledit distributeur depuis une première configuration, dans laquelle agit sur une première surface (11) de ladite membrane la pression régnant à l'intérieur de ladite chambre tandis que la pression atmosphérique agit sur une seconde surface (10) de la membrane, jusque dans une seconde configuration dans laquelle ladite seconde surface est également sollicitée par une force prédéterminée engendrée par un ressort (15), caractérisée par le fait que ledit ressort est disposé à l'intérieur d'un piston (16) qui s'appuie contre ladite seconde surface de ladite membrane et qui est mobile axialement à l'intérieur d'une partie cylindrique d'un couvercle du carter dudit distributeur, ledit piston étant pourvu d'une pluralité de saillies radiales (18), s'appuyant chacune contre une surface de came correspondante (20) ayant un profil hélicoïdal formé sur la surface latérale de ladite partie cylindrique (17), avec un moyen d'actionnement manoeuvrable manuellement pour commander la rotation dudit piston autour de son axe, et par conséquent le mouvement axial dudit piston dans le sens de compression du ressort, afin de commuter ledit distributeur de ladite seconde configuration jusque dans ladite première configuration, ledit moyen d'actionnement comprenant un manchon rotatif (21), coaxial audit piston et interposé entre ce dernier et ladite partie cylindrique du couvercle, ledit manchon étant pourvu de fentes axiales (22), dans chacune desquelles est insérée une desdites saillies, et avec une poignée de manoeuvre (23) qui fait saillie au-dessus dudit couvercle, et ladite valve de demande d'air comprend des moyens d'arrêt (39, 40) pour limiter la rotation du manchon (21) par rapport audit couvercle (2) entre deux positions angulaires prédéterminées qui sont disposées selon l'angle qui est nécessaire pour la rotation dudit manchon, afin de déplacer ledit piston de la position axiale dans laquelle il est trouvé dans ladite seconde configuration jusque dans la position axiale dans laquelle il est trouvé dans ladiute première configuration.

2. Valve de demande d'air selon la revendication 1, caractérisée par le fait que ledit manchon est pourvu d'une paroi inférieure de base (24) qui agit comme un support pour ledit ressort, ainsi que d'un appendice (26), faisant saillie axialement de ladite paroi de base et positionné à l'intérieur dudit ressort pour le centrer axialement.

3. Valve de demande d'air selon une quelconque des revendications précédentes, caractérisée par le fait que ledit couvercle comprend une partie annulaire en relief (32), coaxiale avec ledit trou, disposée autour de lui, et une collerette annulaire (34) s'étendant radialement vers l'extérieur à partir de ladite partie annulaire en relief, ladite poignée dudit élément étant pourvue d'une première paroi plane (35) qui repose sur ladite partie annulaire en relief et sur ladite collerette annulaire, ainsi que d'une seconde paroi cylindrique (36), placée périphériquement sur le côté extérieur de ladite collerette annulaire et disposée autour d'elle, au moins une partie de ladite seconde paroi cylindrique étant pourvue de moyens élastiques de fixation, insérés comme des ressorts dans ladite collerette annulaire, afin ds fixer axialement ladite poignée par rapport à la collerette annulaire.

4. Valve de demande d'air selon la revendication 3, caractérisée par le fait que lesdits moyens élastiques de fixation sont réalisés au moyen de découpures radiales (37), formées dans lesdites première et seconde parois de la poignée, qui rendent élastiquement déformable le secteur (38) de ladite poignée qui est située entre deux découpures contigues, et par l'intermédiaire de dentes radiales (39) prévues sur la surface annulaire de ladite seconde paroi et qui entrent en contact avec ladite collerette annulaire.

5. Valve de demande d'air selon la revendication 1, caractérisée par le fait que lesdits moyens d'arrêt comprennent des protubérances radiales (40) prévues sur ladite partie annulaire en relief du couvercle de façon à coopérer avec lesdites dents radiales (39) de ladite seconde paroi de poignée.

6. Valve de demande d'air selon une quelconque des revendications 3 à 5, caractérisée par le fait qu'il est prévu dans ladite première paroi de poignée (35) au moins une ouverture (41) pour découvrir des parties de la surface de ladite partie annulaire en relief et de ladite collerette annulaire pour fournir une indication visuelle de la position angulaire de la poignée.

7. Valve de demande d'air selon la revendication 6, caractérisée par le fait que ledit couvercle comprend une partie annulaire en relief (33a) qui est alignée avec ladite ouverture (41) pour fournir une indication tactile de ladite position angulaire.

EP 0 126 321 B1

Fig.2

Fig.1

Fig.4

Fig.3

Fig. 5

Fig.6

Fig.7

Fig.8

Fig.9

Fig.10